(19) Europäisches Patentamt
European Patent Office
Office européen des brevets

(11) EP 4 233 676 A1

(12) EUROPEAN PATENT APPLICATION
published in accordance with Art. 153(4) EPC

(43) Date of publication:
30.08.2023 Bulletin 2023/35

(21) Application number: 20958657.7

(22) Date of filing: 21.10.2020

(51) International Patent Classification (IPC):
A61B 1/00 (2006.01)

(52) Cooperative Patent Classification (CPC):
A61B 1/00

(86) International application number:
PCT/JP2020/039522

(87) International publication number:
WO 2022/085104 (28.04.2022 Gazette 2022/17)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicants:
• NEC Corporation
108-8001 Tokyo (JP)
• Olympus Medical Systems Corp.
Tokyo 192-8507 (JP)

(72) Inventors:
• YOSHIOKA, Masaru
Tokyo 108-8001 (JP)

• KAMIJO, Kenichi
Tokyo 108-8001 (JP)
• MANO, Mieko
Tokyo 108-8001 (JP)
• KOBAYASHI, Katsuhiko
Tokyo 108-8001 (JP)
• IKEDA, Yuichi
Hachioji-shi, Tokyo 192-8507 (JP)
• FUJITA, Hiromasa
Hachioji-shi, Tokyo 192-8507 (JP)

(74) Representative: Betten & Resch
Patent- und Rechtsanwälte PartGmbB
Maximiliansplatz 14
80333 München (DE)

(54) **ENDOSCOPE OPERATION ASSISTANCE DEVICE, CONTROL METHOD, COMPUTER READABLE MEDIUM, AND PROGRAM**

(57) An endoscope operation support apparatus (2000) acquires a shape data sequence (50) representing a temporal change of a shape category of an endoscope scope (40). The endoscope operation support apparatus (2000) uses the shape data sequence (50) to determine a target category suitable as a transition destination of the shape category of the endoscope scope (40) or a target position which is a position suitable as a position at which the endoscope scope (40) transitions. The endoscope operation support apparatus (2000) outputs target information (20) that is information regarding the target category or the target position.

Fig. 1

**Description**

**Technical Field**

The present disclosure relates to a method for supporting an operation of an endoscope.

**Background Art**

Apparatuses that support surgery, examination, and the like using an endoscope have been developed. For example, Patent Literature 1 discloses a technology in which an empirical rule of a skilled doctor is programmed and an operation to be performed on an endoscope is presented based on an image obtained from an endoscope camera and a shape of the endoscope.

**Citation List**

**Patent Literature**

Patent Literature 1: Japanese Unexamined Patent Application Publication No. 2019-097661

**Summary of Invention**

**Technical Problem**

The present inventor has studied a new technology for supporting an operation of an endoscope. An object of the present disclosure is to provide a new technology for supporting an endoscope operation.

**Solution to Problem**

According to an aspect of the present disclosure, there is provided an endoscope operation support apparatus including: an acquisition unit configured to acquire a shape data sequence representing a temporal change of a shape category of an endoscope scope inserted into a body; a determination unit configured to determine a target category suitable as a transition destination of the shape category of the endoscope scope or a target position which is a position suitable as a position at which the endoscope scope transitions based on the shape data sequence; and an output unit configured to output target information that is information regarding the target category or the target position.

According to another aspect of the present disclosure, there is provided a control method executed by a computer. The control method includes: an acquisition step of acquiring a shape data sequence representing a temporal change of a shape category of an endoscope scope inserted into a body; a determining step of determining a target category suitable as a transition destination of the shape category of the endoscope scope or a target position which is a position suitable as a position at which the endoscope scope transitions based on the shape data sequence; and an output step of outputting target information that is information regarding the target category or the target position.

According to still another aspect, there is provided a computer readable medium storing a program, the program causing a computer to execute the control method of the present invention.

**Advantageous Effects of Invention**

According to the present disclosure of the present disclosure, a new technology for supporting an endoscope operation is provided.

**Brief Description of Drawings**

Fig. 1 is a diagram illustrating an overview of an operation of an endoscope operation support apparatus according to a first example embodiment.
Fig. 2 is a block diagram illustrating a functional configuration of the endoscope operation support apparatus according to the first example embodiment.
Fig. 3 is a block diagram illustrating a hardware configuration of a computer that implements the endoscope operation support device.

Fig. 4 is a diagram illustrating a specific example of a use environment of an endoscope operation support device. For example, endoscope operation

Fig. 5 is a flowchart illustrating a flow of processing executed by the endoscope operation support apparatus according to the first example embodiment.

Fig. 6 is a diagram illustrating a configuration of a shape data sequence in a table format.

Fig. 7 is a diagram illustrating a state transition model of an insertion method of an endoscope scope.

Fig. 8 is a diagram conceptually illustrating a method of calculating accuracy using Equation (1).

Fig. 9 is a diagram illustrating a screen including target information.

Fig. 10 is a diagram illustrating target information including a plurality of shape categories determined as candidates of a target category.

Fig. 11 is a diagram illustrating target information including a target position.

Fig. 12 is a diagram illustrating target information representing a shape of the endoscope scope in a three-dimensional image.

Fig. 13 is a diagram illustrating target information in which a shape of an endoscope scope corresponding to a target category is represented by a three-dimensional image.

## Example Embodiment

Hereinafter, example embodiments of the present disclosure will be described in detail with reference to the drawings. In the drawings, the same or corresponding elements are denoted by the same reference numerals, and repeated description is omitted as necessary for clarity of description. In addition, unless otherwise described, predetermined values such as predetermined values and thresholds are stored in advance in a storage device accessible from an apparatus using the values.

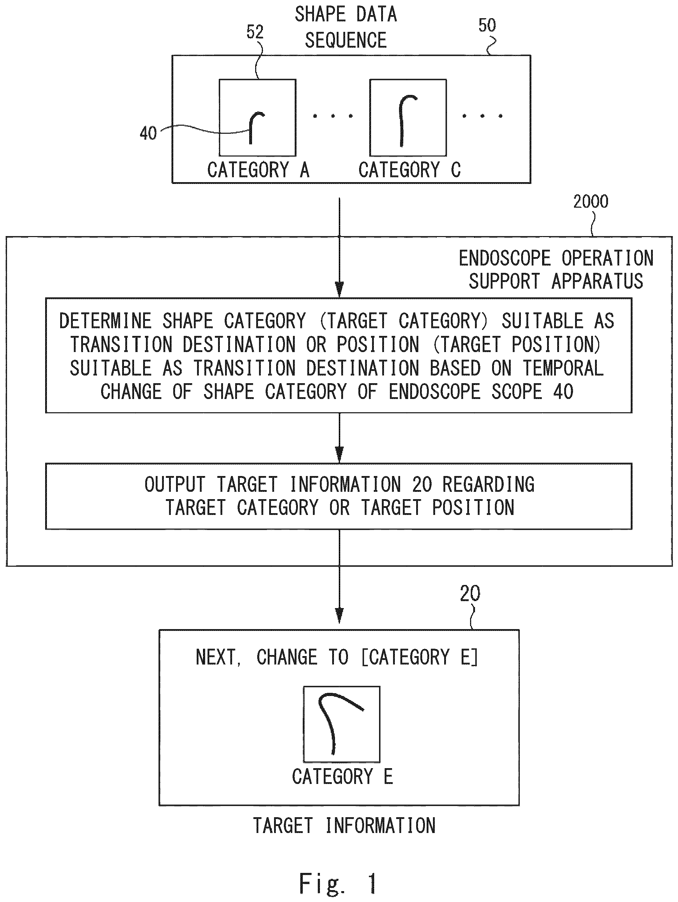

Fig. 1 is a diagram illustrating an overview of an operation of an endoscope operation support apparatus 2000 according to a first example embodiment. Here, Fig. 1 is a diagram for facilitating understanding of the overview of the endoscope operation support apparatus 2000, and the operation of the endoscope operation support apparatus 2000 is not limited to that illustrated in Fig. 1.

In surgery, examination, or the like using an endoscope (hereinafter, endoscopic surgery or the like), an operator of an endoscope scope 40 inserts the endoscope scope 40 into a body while variously changing a shape of the endoscope scope 40. Here, it is assumed that shapes that can be taken by the endoscope scope 40 are classified into a plurality of categories in advance. Such classification of the shape of the endoscope scope 40 is referred to as a shape category. For example, as the shape category, various categories such as "straight line ", "small curve", "large curve", and "abdomen protrusion" can be defined.

How the shape category of the endoscope scope 40 should be changed may vary depending on how the shape category of the endoscope scope 40 has changed. Therefore, the endoscope operation support apparatus 2000 acquires a shape data sequence 50 representing a temporal change of the shape category of the endoscope scope 40 inserted into the body, and uses the shape data sequence 50 to determine the shape category to which the endoscope scope 40 should transition. The shape category (that is, a shape category appropriate as a transition destination) determined in this manner is referred to as a target category.

The endoscope operation support apparatus 2000 outputs target information 20 regarding the determined target category. For example, the target category indicates a name of a shape category to be taken next by the endoscope scope 40. For example, in the example of Fig. 1, the target information 20 includes a message representing that the target category is a "category E" and an image representing the shape of the endoscope scope 40 classified into the category E.

However, the target information 20 may indicate a target position that is an appropriate position as a transition destination of the endoscope scope 40, in addition to the target category or instead of the target category.

<Example of Advantageous Effect>

As described above, the endoscope operation support apparatus 2000 according to the present example embodiment determines the target category or the target position using the shape data sequence 50 representing the temporal change in the shape category of the endoscope scope 40. That is, it is determined to which shape category it is appropriate to transition the endoscope scope 40 or to which position the endoscope scope 40 should transition. Then, the endoscope operation support apparatus 2000 outputs the target information 20 regarding the target category or the target position.

By using the target information 20, the operator or the like of the endoscope scope 40 can grasp how the shape of the endoscope scope 40 should be changed and which position of the endoscope scope 40 should be changed. Here, when the target shape category and the target position are known, it is possible to determine what operation should be performed on the endoscope scope 40. Therefore, the operator or the like of the endoscope scope 40 can grasp an appropriate operation to be performed on the endoscope scope 40.

Hereinafter, the endoscope operation support apparatus 2000 according to the present example embodiment will be described in more detail.

<Example of Functional Configuration>

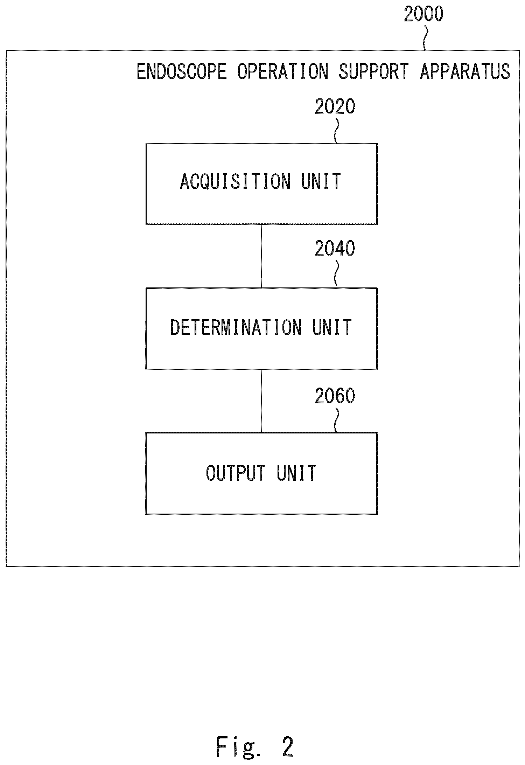

Fig. 2 is a block diagram illustrating a functional configuration of the endoscope operation support apparatus 2000 according to the first example embodiment. The endoscope operation support apparatus 2000 includes an acquisition unit 2020, a determination unit 2040, and an output unit 2060. The acquisition unit 2020 acquires the shape data sequence 50. The determination unit 2040 determines the target category or the target position using the shape data sequence 50. The output unit 2060 outputs the target information 20 regarding the target category or the target position.

<Example of Hardware Configuration>

Each functional component of the endoscope operation support apparatus 2000 may be realized by hardware (for example, a hard-wired electronic circuit or the like) that realizes each functional component, or may be realized by a combination of hardware and software (for example, a combination of an electronic circuit and a program for controlling the electronic circuit or the like). Hereinafter, a case where each functional component of the endoscope operation support apparatus 2000 is realized by a combination of hardware and software will be further described.

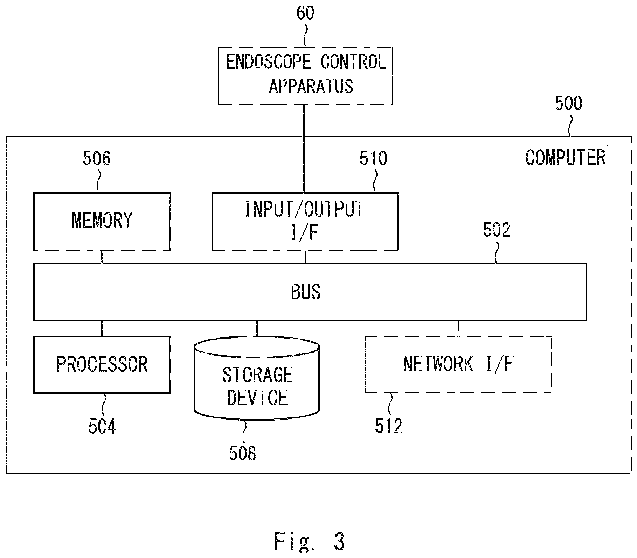

Fig. 3 is a block diagram illustrating a hardware configuration of a computer 500 that implements the endoscope operation support apparatus 2000. The computer 500 is any computer. For example, the computer 500 is a stationary computer such as a personal computer (PC) or a server machine. In another example, the computer 500 is a portable computer such as a smartphone or a tablet terminal. The computer 500 may be a special-purpose computer designed to realize the endoscope operation support apparatus 2000, or may be a general-purpose computer.

For example, by installing a predetermined application in the computer 500, each function of the endoscope operation support apparatus 2000 is realized in the computer 500. The application includes a program for realizing the functional component of the endoscope operation support apparatus 2000. The installation of the application can be realized by copying the program from a recording medium (for example, a USB memory, a DVD, or the like) in which the program is stored or downloading the program from a server that manages a storage device in which the program is stored.

The computer 500 includes a bus 502, a processor 504, a memory 506, a storage device 508, an input/output interface 510, and a network interface 512. The bus 502 is a data transmission path for the processor 504, the memory 506, the storage device 508, the input/output interface 510, and the network interface 512 to transmit and receive data to and from each other. However, a method of connecting the processor 504 and the like to each other is not limited to the bus connection.

The processor 504 is various processors such as a central processing unit (CPU), a graphics processing unit (GPU), or a field-programmable gate array (FPGA). The memory 506 is a primary storage device realized by using a random access memory (RAM) or the like. The storage device 508 is a secondary storage device realized by using a hard disk, a solid state drive (SSD), a memory card, a read only memory (ROM), or the like.

The input/output interface 510 is an interface for connecting the computer 500 and an input/output device. For example, an endoscope control apparatus 60 to be described later is connected to the input/output interface 510. In another example, an input device such as a keyboard and an output device such as a display device are connected to the input/output interface 510.

The network interface 512 is an interface for connecting the computer 500 to a network. The network may be a local area network (LAN) or a wide area network (WAN).

The storage device 508 stores a program (program for realizing the above-described application) for realizing each functional component of the endoscope operation support apparatus 2000. The processor 504 implements each functional component of the endoscope operation support apparatus 2000 by reading and executing the program in the memory 506. The program is stored in the storage device 508, for example, by being copied from a storage medium (for example, a USB memory) in which the program is stored.

The endoscope operation support apparatus 2000 may be realized by one computer 500 or may be realized by a plurality of computers 500. In the latter case, the configurations of the computers 500 do not need to be the same, and can be different from each other.

<Example of Use Environment of Endoscope Operation Support Apparatus 2000>

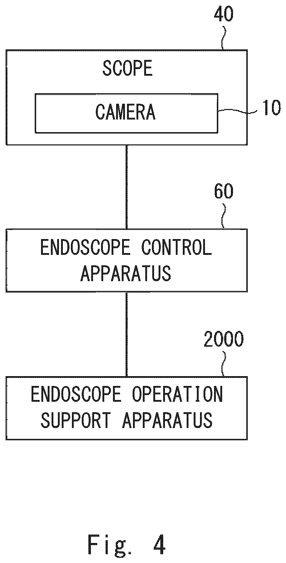

Fig. 4 is a diagram illustrating a specific example of a use environment of the endoscope operation support apparatus 2000. For example, the endoscope operation support apparatus 2000 is used together with the endoscope scope 40 and the endoscope control apparatus 60. The endoscope scope 40 is a scope to be inserted into the body, and is provided with a camera 10. By viewing video data generated by the camera 10, the state in the body can be visually recognized.

The endoscope control apparatus 60 is a control apparatus used when an endoscopic surgery or the like is performed using the endoscope scope 40. For example, the endoscope control apparatus 60 generates video data to be viewed, by performing various processing on video data obtained from a camera provided in the endoscope scope 40, and outputs the generated video data. Examples of processing on the video data include processing of adjusting color or brightness of the video data and processing of superimposing various types of information on the video data.

For example, the endoscope operation support apparatus 2000 acquires various types of information via the endoscope control apparatus 60. For example, the endoscope operation support apparatus 2000 acquires still image data or video data (hereinafter, these are collectively referred to as image data) generated by the camera 10 from the endoscope control apparatus 60.

Note that the data obtained from the endoscope scope 40 is not limited to the image data generated by the camera 10. For example, the endoscope scope 40 may be provided with elements for obtaining three-dimensional coordinates for each of a plurality of locations of the endoscope scope 40. In this case, the endoscope control apparatus 60 determines the three-dimensional coordinates of each of the plurality of locations of the endoscope scope 40 by using the element. By using the three-dimensional coordinates for the plurality of locations of the endoscope scope 40 obtained in this manner, it is possible to grasp the shape of the endoscope scope 40.

<Flow of Processing>

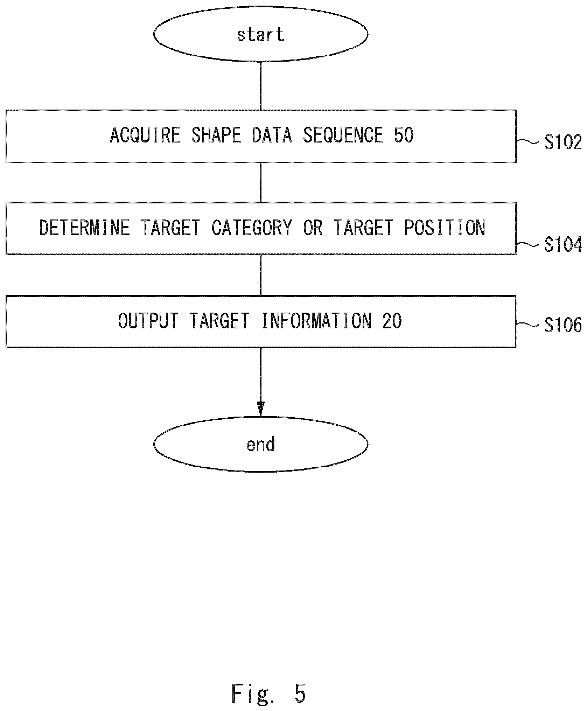

Fig. 5 is a flowchart illustrating a flow of processing executed by the endoscope operation support apparatus 2000 according to the first example embodiment. The acquisition unit 2020 acquires the shape data sequence 50 (S102). The determination unit 2040 determines the target category or the target position using the shape data sequence 50 (S104). The determination unit 2040 outputs the target information 20 regarding the determined target category or target position (S106).

<Shape Data sequence 50>

The shape data sequence 50 is data representing the shape of the endoscope scope 40 in time series. For example, the shape data sequence 50 indicates the shape of the endoscope scope 40 at each of a plurality of time points. In the shape data sequence 50, the shape of the endoscope scope 40 is represented by one of a plurality of determined shape categories.

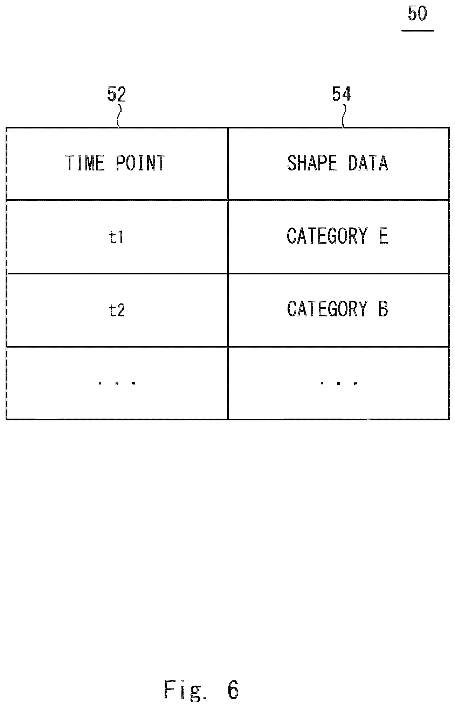

Fig. 6 is a diagram illustrating a configuration of the shape data sequence 50 in a table format. The shape data sequence 50 in Fig. 6 includes a time point 51 and shape data 52. Each record in the shape data sequence 50 indicates that, at a time point indicated by the time point 51, the shape of the endoscope scope 40 was in the shape category indicated in the shape data 52. The time point 51 may be represented by time or may be represented by other than time. In the latter case, for example, the time point 51 indicates a relative numerical value assigned to each piece of shape data 52 in chronological order, such as a sequence number.

Which shape category the shape of the endoscope scope 40 belongs to can be determined using, for example, the three-dimensional coordinates of each of a plurality of specific locations of the endoscope scope 40. As described above, for example, the three-dimensional coordinates of each of the plurality of locations of the endoscope scope 40 can be determined using the elements provided at those locations.

For example, an identification model (hereinafter, shape identification model) is used to determine the shape category. The shape identification model can be realized by various types of models such as a neural network or a support vector machine. The shape identification model is trained in advance so as to output a label representing a shape category of the endoscope scope 40 in response to input of three-dimensional coordinates of each of a plurality of specific locations of the endoscope scope 40. For each of a plurality of time points, by inputting the three-dimensional coordinates of each of a plurality of specific locations of the endoscope scope 40 at that time point to the shape identification model, it is possible to determine the shape category of the endoscope scope 40 at that time point. Note that training of the shape identification model can be performed by using training data including a combination of "three-dimensional coordinates of each of a plurality of specific locations of the endoscope scope 40, and a label representing a ground-truth shape category".

<Acquisition of Shape Data sequence 50: S102>

The acquisition unit 2020 acquires the shape data sequence 50 (S102). There are various methods for the acquisition unit 2020 to acquire the shape data sequence 50. For example, the acquisition unit 2020 acquires the shape data sequence 50 stored in a storage apparatus accessible from the acquisition unit 2020 by accessing the storage device. The storage apparatus may be provided inside the endoscope operation support apparatus 2000 or may be provided outside the endoscope operation support apparatus 2000. In another example, the acquisition unit 2020 may acquire the shape data sequence 50 by receiving the shape data sequence 50 transmitted from another device.

Here, in the shape data sequence 50, the shape of the endoscope scope 40 is represented by a shape category. Therefore, in order to generate the shape data sequence 50, it is necessary to perform processing of determining the shape category of the endoscope scope 40 at each time point. This processing may be performed by the endoscope operation support apparatus 2000 or may be performed by an apparatus other than the endoscope operation support apparatus 2000. In the former case, the acquisition unit 2020 acquires the shape data sequence 50 generated inside the endoscope operation support apparatus 2000. For example, in this case, the shape data sequence 50 is stored in a storage apparatus inside the endoscope operation support apparatus 2000. Meanwhile, in a case where the shape data sequence 50 is generated by an apparatus other than the endoscope operation support apparatus 2000, for example, the acquisition unit 2020 acquires the shape data sequence 50 by accessing the apparatus that has generated the shape data sequence 50 or receives the shape data sequence 50 transmitted from the device.

Here, since the endoscope scope 40 is inserted into the body while changing the shape, the target category changes with time. Therefore, for example, it is preferable that the endoscope operation support apparatus 2000 acquires the shape data sequence 50 representing the temporal change of the shape data 52 in a period of a predetermined length (hereinafter, the unit period), and determines the target category corresponding to the shape data sequence 50. In this case, the endoscope operation support apparatus 2000 may acquire the shape data sequence 50 divided for each unit period in advance, or may acquire a plurality of pieces of shape data 52 not divided for each unit period.

In the latter case, for example, the acquisition unit 2020 obtains the shape data sequence 50 for each unit period by dividing the plurality of pieces of acquired shape data 52 for each unit period in chronological order. However, the unit periods adjacent to each other may partially overlap each other. For example, in a case where the length of the unit period is 10 and the length of the overlapping period is 4, the first shape data sequence 50 includes the first to tenth shape data 52, and the second shape data sequence 50 includes the seventh to sixteenth shape data 52.

identification of Target Category: S104>

The determination unit 2040 determines the target category using the shape data sequence 50 (S104). There are various specific methods for determining the target category using the shape data sequence 50. Hereinafter, some specific methods will be exemplified. A method of determining the target position will be described later.

<<Method Using Identification Model>>

For example, the determination unit 2040 determines the target category using the identification model. For example, the identification model is trained so as to output likelihood that each shape category is the target category in response to the input of the shape data sequence 50. Hereinafter, this identification model is referred to as a target category identification model. For example, the target category identification model is realized by using a recurrent nueral network (RNN) which is a neural network that handles time-series data. However, the type of the model used for realizing the target category identification model is not limited to the RNN, and any type of model that can handle the time-series data can be used.

The training of the target category identification model is performed using training data including a combination of "data representing temporal change in shape category of endoscope scope, and ground-truth output data". The ground-truth output data is, for example, data representing the maximum likelihood (for example, 1) for the shape category to be treated as the target category and representing the minimum likelihood (for example, 0) for the other shape categories.

The determination unit 2040 determines likelihood that each shape category is the target category by inputting the shape data sequence 50 to the target category identification model. Then, the determination unit 2040 determines the target category based on the determined likelihood.

Various methods can be employed as a method of determining the target category based on the likelihood computed for each shape category. For example, the determination unit 2040 determines the shape category having the maximum likelihood as the target category. In another example, the determination unit 2040 determines, as the target category, one or more shape categories whose likelihood is equal to or more than a threshold or a plurality of shape categories whose order of the likelihood is equal to or lower than a predetermined order.

Note that the determination unit 2040 may treat the plurality of shape categories whose likelihood is equal to or more than the threshold or the plurality of shape categories whose order of the likelihood is equal to or lower than the predetermined order as candidates of the target category, and determine the target category from the candidate shape categories. There are various methods for determining the target category from the candidate shape categories. For example, a priority is assigned to each shape category in advance. In this case, the determination unit 2040 determines a shape category having the highest priority among the candidate shape categories as the target category.

Here, which shape category should be prioritized may vary depending on the situation. For example, there are various insertion methods used to insert the endoscope scope 40 (described in detail below), and the priority of the shape category may vary for each insertion method. Therefore, for example, the priority of the shape category is determined in advance for each insertion method. Specifically, insertion method information that is information regarding the insertion method is stored in advance in a storage device accessible from the determination unit 2040. In the insertion method information, the priority of each shape category is determined for each insertion method.

In this case, the determination unit 2040 determines an insertion method used for insertion of the endoscope scope 40 using a method to be described later. The determination unit 2040 determines the priority of each shape category using the insertion method information regarding the determined insertion method. Then, the determination unit 2040 determines a determined shape category having the highest priority among the candidate shape categories as the target category. This makes it possible to determine an appropriate target category according to the insertion method being used.

In another example, which shape category should be prioritized may be different for each operator of the endoscope scope 40. Therefore, the priority of the shape category may be determined for each operator of the endoscope scope 40. For example, operator information that is information regarding the operator is stored in advance in a storage device accessible from the determination unit 2040. In the operator information, the priority of each shape category is determined for each operator.

In this case, the determination unit 2040 acquires the operator information regarding the operator operating the endoscope scope 40, thereby determining the priority of each shape category for the operator. Then, the determination unit 2040 determines a determined shape category having the highest priority among the candidate shape categories as the target category. Accordingly, it is possible to determine an appropriate target category according to the feature of the operator.

There are various methods for determining association between the operator and the priority of the shape category. For example, the association between the operator and the priority of the shape category can be determined using a record (video data or the like) of past endoscopic surgery or the like performed by the operator. Specifically, it is conceivable that the video data is analyzed to count the number of occurrences of each shape category, and the shape category having a larger number of occurrences has a higher priority order. In another example, the priority of each shape category may be determined for the operator by manual setting by the operator or a supervisor of the operator.

In another example, which shape category should be prioritized may vary depending on the technical level of the operator of the endoscope scope 40. Therefore, the priority of each shape category may be determined for each predetermined technical level (for example, five levels from level 1 to level 5, and the like). For example, technical level information that is information in which the technical level and priority of each shape category are associated with each other is stored in advance in a storage device accessible from the determination unit 2040. The operator information described above includes information representing the technical level of each operator.

In this case, the determination unit 2040 determines the technical level of the operator using the operator information regarding the operator of the endoscope scope 40. In addition, the determination unit 2040 determines the priority of each shape category by acquiring the technical level information corresponding to the determined technical level. Then, the determination unit 2040 determines a determined shape category having the highest priority among the candidate shape categories as the target category. This makes it possible to determine an appropriate target category according to the technical level of the operator.

There are various methods for determining the technical level of each operator. For example, a numerical range of the years of experience is divided into predetermined stages. In this case, the technical level of the operator is determined by the numerical range to which the years of experience of the operator belong. In another example, the technical level of the operator may be determined by evaluating the technical level of the operator using a history (video data or the like) of past endoscopic surgery or the like performed by the operator. In another example, the technical level of the operator may be determined by manual setting by the operator or a supervisor of the operator.

In another example, the target category may be determined using information regarding a person (hereinafter, subject) into which the endoscope scope 40 is inserted. For example, a place where there is a possibility that adhesion occurs in the body can be determined based on the past surgical history of the subject. Then, the shape category to be selected may be different depending on the presence or absence of adhesion. Therefore, information in which the type of surgery or the like performed in the past is associated with the priority of each shape category is stored in advance in a storage device accessible from the determination unit 2040. The determination unit 2040 determines surgery or the like performed on the subject in the past by acquiring information representing a history of surgery or the like in the past for the subject. Furthermore, the determination unit 2040 acquires the priority of each shape category associated with the determined surgery or the like, and determines the target category from the candidate shape category using the priority. This makes it possible to determine an appropriate target category in consideration of the influence of past surgery or the like.

Furthermore, the priority of the shape category may be determined based on an operation history of the endoscope scope 40 in surgery or the like having the same content performed on another person in the past. Even in this case, the information in which the type of surgery or the like is associated with the priority of each shape category is stored in advance in the storage device accessible from the determination unit 2040. For surgery or the like performed on the subject, the determination unit 2040 acquires the priority of each shape category associated with the surgery or the like. Then, the determination unit 2040 determines the target category from the candidate shape categories using the acquired priority. As a result, it is possible to determine an appropriate target category based on experience of past surgery or the like.

The determination unit 2040 may determine a priority order for the candidate shape category instead of determining one of the candidate shape categories as the target category. For example, a shape category with higher likelihood is assigned a higher priority order. In another example, by determining the priority of the shape category by various methods described above, a shape category having a higher priority may be assigned a higher priority order. The determined priority order can be used, for example, when emphasis based on the priority order is performed on a plurality of target categories in the target information 20 as described later.

Data other than the shape data sequence 50 may be further input to the target category identification model. Examples of the data other than the shape data sequence 50 include still image data or video data obtained from the camera 10, data representing a motion of the operator of the endoscope scope 40 (for example, time-series data of acceleration obtained from an acceleration sensor attached to the hand of the operator, and the like), information regarding the operator of the endoscope scope 40 (the technical level of the operator and the priority of each shape category defined for the operator), and the like. Furthermore, the three-dimensional coordinates of each of the plurality of locations of the endoscope scope 40 may be further input to the target category identification model. When these data are used, the same type of data is also included in the training data of the target category identification model. In this way, the target category identification model is trained to compute likelihood by further using data other than the shape data sequence 50.

The target category identification model may output a label representing the target category instead of outputting the likelihood for each shape category. In this case, for example, the target category identification model outputs a label representing the shape category for which the maximum likelihood is computed as a label of the target category. The determination unit 2040 can determine the target category by the label output from the target category identification model.

<<Method Using State Transition Model>>

For example, the determination unit 2040 determines an insertion method used for insertion of the endoscope scope 40, and determines the target category based on a state transition model defined for the insertion method. Here, a plurality of insertion methods may be used to insert the endoscope scope. Examples of the insertion method include a shaft retention and shortening method, a push method, and a method of forming an $\alpha$ loop.

The temporal change in shape of the endoscope scope 40 may vary for each insertion method used. Therefore, by determining the insertion method used to insert the endoscope scope 40, it is possible to determine how the shape of the endoscope scope 40 should change in the future. Therefore, the determination unit 2040 determines the target category by determining the insertion method used to insert the endoscope scope 40. Note that a specific method of determining the insertion method will be described later.

For example, for each of a plurality of insertion methods, a state transition model representing a temporal change pattern of the shape category of the endoscope scope 40 in a case where the insertion method is used is determined and stored in advance in a storage apparatus accessible from the determination unit 2040. Then, the determination unit 2040 determines the target category using the state transition model of the insertion method used for the insertion of the endoscope scope 40.

The shape of the endoscope scope 40 preferably transitions according to a state transition model of an insertion method used for insertion of the endoscope scope 40. Therefore, for example, the determination unit 2040 determines a shape category positioned next to the last shape category in the shape data sequence 50 as the target category in the state transition model of the insertion method used for insertion of the endoscope scope 40.

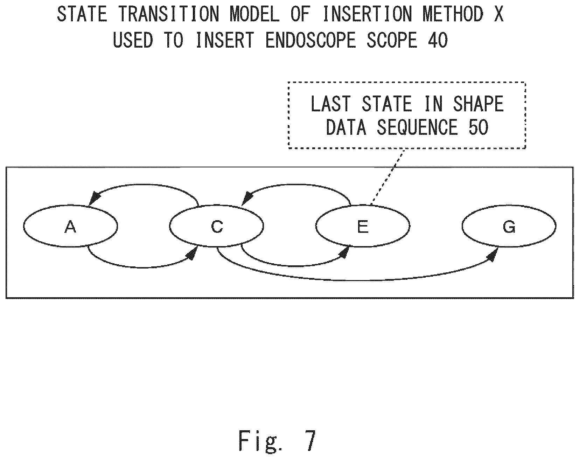

A specific example of a method of determining the target category using the state transition model will be described. Fig. 7 is a diagram illustrating a state transition model of the insertion method of the endoscope scope 40. Here, it is assumed that the determination unit 2040 determines that an insertion method X is used to insert the endoscope scope 40 using the shape data sequence 50. In addition, it is assumed that a state transition model of the insertion method X is illustrated in Fig. 7. Then, it is assumed that the last shape category in the shape data sequence 50 is the category E.

The determination unit 2040 determines a category to which transition from a category E is made in the state transition model of the insertion method X as the target category. In this regard, in the state transition model of the insertion method X illustrated in Fig. 7, the destination of transition from the category E is a category C. Therefore, the determination unit 2040 determines the category C as the target category.

In the state transition model, a plurality of shape categories to which it is possible to transition from one shape category may be defined. Therefore, in the state transition model of the insertion method used, there may be a plurality of shape categories to which it is possible to transition from the last shape category of the shape data sequence 50. For example, in the example of Fig. 7, there are three categories A, E, and G as the shape categories to which it is possible to transition from the category C. Therefore, when the last shape category in the shape data sequence 50 is the category C, there are three shape categories to which it is possible to transition.

Therefore, for example, the determination unit 2040 treats a plurality of shape categories to which it is possible to transition from the last shape category of the shape data sequence 50 as candidates of the target category. For example, the determination unit 2040 determines one of these candidate shape categories to be treated as the target category.

There are various methods for determining the target category from the candidate shape categories. For example, in the state transition model, in a case where a transition can be made from a certain state to a plurality of states, it is assumed that a transition probability is assigned to each transition. In this case, for example, the determination unit 2040 determines a candidate shape category having the maximum transition probability as the target category.

In another example, similarly to the case of determining the target category using the target category identification model, the priority may be determined for the shape category. In this case, as described above, the determination unit 2040 determines the shape category having the highest priority among the candidate shape categories as the target category.

In addition, instead of determining one target category, all or some of the candidate shape categories may be determined as the target category. In a case where a part of the candidate shape categories is treated as the target category, for example, the determination unit 2040 determines, as the target category, the shape category having the transition probability equal to or more than the threshold, the shape category whose order of the transition probability is less than or equal to a predetermined order, the shape category whose order of the priority is less than or equal to the predetermined order, or the like.

In addition, the determination unit 2040 may determine the priority order with respect to the candidate shape category. For example, a shape category having a higher transition probability can be assigned a higher priority order, or a shape category having a higher priority can be assigned a higher priority order. The method of using the priority order is as described above.

<<<Method in Consideration of Change in Insertion Method>>>

It may be preferable to change the insertion method used to insert the endoscope scope 40. For example, in a case where the state of the endoscope scope 40 stays for a long time, it is considered preferable to change to another insertion method. In another example, when an operator with a low technical level (for example, a young doctor) uses a difficult insertion method requiring a high technical level, it is considered preferable to use a simpler insertion method.

Therefore, for example, the determination unit 2040 may determine whether or not it is necessary to change the insertion method, and when it is necessary to change the insertion method, the target category may be determined using a state transition model of a new insertion method. For example, the determination unit 2040 determines whether or not it is necessary to change the insertion method, and determines another insertion method (hereinafter, changed insertion method) to be changed from the current insertion method in a case where it is determined that it is necessary to change the insertion method. Then, the determination unit 2040 determines the target category by comparing the state transition model of the changed insertion method with the shape data sequence 50. The method of determining the target category using the state transition model and the shape data sequence 50 is as described above.

The determination as to whether or not the insertion method needs to be changed can be made using a predetermined condition determined in advance. As the predetermined condition, various conditions can be used. For example, the predetermined condition is a condition such as "a residence time is equal to or more than a threshold" or "the technical level associated with the current insertion method is higher than the technical level defined for the operator of the endoscope scope 40". When the predetermined condition is satisfied, it is determined that the insertion method needs to be changed. Meanwhile, when the predetermined condition is not satisfied, it is determined that there is no need to change the insertion method.

As the residence time, for example, a time during which the same insertion method continues, a time during which a transition loop continues on the state transition model, and the like can be handled. Here, a time required to complete one insertion method may vary for each insertion method. Therefore, in the insertion method definition information described above, a time to be treated as the threshold of the residence time may be determined for each insertion method. In this case, when determining the currently used insertion method, the determination unit 2040 acquires the threshold of the residence time associated with the insertion method. Then, the determination unit 2040 determines whether or not a duration of the currently used insertion method is equal to or more than the threshold, and when the duration is equal to or more than the threshold, it is determined that the insertion method needs to be changed.

When the technical level is handled as the predetermined condition, each insertion method and the technical level necessary for use of the insertion method are associated with each other in the insertion method definition information. The operator information described above includes information representing the technical level of each operator. Using the insertion method definition information and the operator information, the determination unit 2040 determines whether or not the technical level necessary for the currently used insertion method exceeds the technical level of the operator of the endoscope scope 40. Then, in a case where the technical level required for the currently used insertion method exceeds the technical level of the operator of the endoscope scope 40, the determination unit 2040 determines that the insertion method should be changed to another insertion method.

In a case where it is determined that the insertion method needs to be changed, the determination unit 2040 determines the changed insertion method. For example, in the insertion method definition information, another insertion method that can substitute the insertion method is defined for each insertion method. For example, it is assumed that insertion methods A and B exist as insertion methods for advancing the endoscope scope from a first site to a second site in a body. In this case, the insertion method B can be defined as an alternative insertion method of the insertion method A. Similarly, the insertion method A can be defined as an alternative insertion method of the insertion method B. The determination unit 2040 uses the insertion method definition information to determine the insertion method associated with the currently used insertion method, and determines the determined insertion method as the changed insertion method.

However, when it is determined that the insertion method needs to be changed because the currently used insertion method exceeds the technical level of the operator, it is considered that it is not preferable to change the insertion method to a more difficult insertion method. Therefore, for example, when it is determined that the technical level required for the currently used insertion method exceeds the technical level of the operator of the endoscope scope 40, the determination unit 2040 determines whether or not the technical level required for another insertion method associated with the currently used insertion method is lower than the technical level required for the currently used insertion method. When the technical level required by another insertion method associated with the currently used insertion method is lower than the technical level required by the currently used insertion method, the determination unit 2040 determines the another insertion method as the changed insertion method. Meanwhile, when the technical level required by another insertion method associated with the currently used insertion method is equal to or higher than the technical level required by the currently used insertion method, the determination unit 2040 determines not to change the insertion method.

In addition, instead of making such a determination, when another insertion method that can replace the insertion method is associated with the insertion method, the latter insertion method may always adopt an insertion method that can be used at a technical level lower than the technical level required for the former insertion method. In this way, when the insertion method associated with the currently used insertion method is obtained from the insertion method definition information, the technical level required for the obtained insertion method is necessarily lower than the technical level required for the currently used insertion method.

<<Combination of Plurality of Methods>>

The determination unit 2040 may determine the target category using both the target category identification model and the state transition model of the currently used insertion method. For example, the determination unit 2040 determines a plurality of shape categories as candidates of the target category in both the method using the target category identification model and the method using the state transition model. Then, the determination unit 2040 determines, as the target category, a shape category that is an overlap between the candidates obtained by the two methods (a shape category obtained as a candidate of the target category by both methods).

In another example, the determination unit 2040 treats a plurality of shape categories that can transition from the last shape category of the shape data sequence 50 in the state transition model of the currently used insertion method as the candidate shape categories. The determination unit 2040 obtains a transition probability from the state transition model for each candidate shape category. In addition, the determination unit 2040 obtains the likelihood output from the target category identification model for each candidate shape category. Further, the determination unit 2040 computes, for each candidate shape category, a statistical value (for example, an average value or a maximum value) of the transition probability obtained from the state transition model and the likelihood obtained from the target category identification model. Then, the determination unit 2040 determines the target category using the statistical value. For example, the determination unit 2040 determines a shape category having the maximum statistical value as the target category. In another example, the determination unit 2040 may treat any one of the candidate shape categories as a target category and perform prioritization based on the statistical value.

In another example, the determination unit 2040 may determine the target category in each of the target category identification model and the state transition model. For example, it is assumed that the target category determined using the target category identification model is the category C, and the target category determined using the state transition model is the category E. In this case, the determination unit 2040 determines the category C and the category E as the target categories. In this case, the target information 20 preferably indicates a determining method and the target category determined by the determining method in association with each other. For example, in the above-described example, by setting "Identification model: Category C, state transition model: Category E" or the like, it is possible to indicate association between the determining method and the target category determined by the determining method.

< Method for Determining Insertion Method >

A method for determining an insertion method currently used will now be described. Various methods can be employed for this method. For example, the determination unit 2040 uses the shape data sequence 50 to compute, for each of a plurality of possible insertion methods, the likelihood of the insertion method being used. Then, the determination unit 2040 determines the insertion method being used from the plurality of insertion methods based on the likelihood computed for each insertion method. Note that a method of computing the likelihood will be described later.

Various methods may be employed to determine the insertion method being used based on the likelihood computed for each insertion method. For example, the determination unit 2040 determines the insertion method with the maximum likelihood as the insertion method being used. In another example, the determination unit 2040 determines the plurality of candidates of the insertion method being used based on the likelihood computed for each insertion method, and determines the insertion method being used from the plurality of candidates. For example, the determination unit 2040 determines the top n (n > 1) insertion methods with high likelihood as the candidate of the insertion method being used. In another example, the determination unit 2040 determines an insertion method whose likelihood is equal to or more than a threshold as the candidate of the insertion method being used.

Various methods can be employed as a method of determining the insertion method being used from a plurality of candidates. For example, a priority is assigned in advance to each of the plurality of insertion methods. In this case, for example, the determination unit 2040 determines an insertion method having the highest priority among the candidate insertion methods as the insertion method being used. In another example, the determination unit 2040 corrects the likelihood by multiplying the computed likelihood by a weight based on the priority for each candidate insertion method. Note that the weight based on the priority is determined in advance as a larger value as the priority is higher. The determination unit 2040 determines an insertion method having the maximum corrected likelihood as the insertion method being used.

In another example, important shape categories or transitions between important shape categories (permutations of two or more shape categories) are determined in advance for each of the plurality of insertion methods. In this case, the determination unit 2040 determines an insertion method in which an important shape category defined for the insertion method or a transition between important shape categories is included in the shape data sequence 50 among the insertion methods determined as the candidates as the insertion method being used.

For example, it is assumed that insertion methods X and Y are determined as candidates based on the likelihood. In addition, it is assumed that an important shape category defined for the insertion method X is B, and an important shape category defined for the insertion method Y is C. Then, it is assumed that the time series of the shape data 52 indicated by the shape data sequence 50 is "A, C, D, C, A". In this case, the shape data sequence 50 does not include the shape category B, which is an important shape category in the insertion method X, while including the shape category C, which is an important shape category in the insertion method Y. Therefore, the determination unit 2040 determines the insertion method Y as the insertion method being used.

It is assumed that there are a plurality of insertion methods in which the shape category or the transition between the shape categories that are important as described above are included in the shape data sequence 50. In this case, for example, the determination unit 2040 determines an insertion method with high computed likelihood among the plurality of insertion methods as the insertion method being used. In another example, the determination unit 2040 may determine, as the insertion method being used, an insertion method in which more important shape categories or transitions between shape categories are included in the shape data sequence 50.

The determination unit 2040 may use the information regarding the operator of the endoscope scope 40 to determine the insertion method being used from the candidate insertion methods. For example, in the operator information, an insertion method that can be used by each operator is described. The determination unit 2040 determines, as the insertion method being used, an insertion method defined as the insertion method that can be used by the operator of the endoscope scope 40 among the candidate insertion methods. Note that, in a case where there are a plurality of candidate insertion methods defined as the insertion methods that can be used by the operator of the endoscope scope 40, for example, the determination unit 2040 determines the insertion method being used based on the likelihood, the height of the priority described above, and the like.

In another example, information representing the technical level of the operator may be used as the information regarding the operator of the endoscope scope 40. In this case, a technical level necessary for each insertion method is determined in the insertion method definition information. The determination unit 2040 determines the insertion method being used from the plurality of candidates based on the relationship between the technical level of the operator and the insertion method.

The determination unit 2040 may determine the insertion method being used from the candidate insertion methods by using image data obtained from the camera 10 provided in the endoscope scope 40. For example, the determination unit 2040 extracts features from the image data obtained from the camera 10, and determines an insertion method matching the features among the candidate insertion methods as the insertion method being used. Features extracted from the image data include, for example, a phenomenon (referred to as a red ball) in which the entire image becomes red due to pressing of a tip of the endoscope scope 40 against the wall of the internal organs, characteristic movement of the screen or operation of the endoscope scope 40, and the like.

The determination unit 2040 may compute the likelihood that each insertion method is used in each of a plurality of methods (for example, a method using an identification model and a method using a state transition model, which will be described later), and determine the insertion method being used based on the computation result. For example, the determination unit 2040 computes a statistical value (for example, an average value or a maximum value) of the likelihood computed by each of the plurality of methods for each insertion method, and determines the insertion method having the maximum statistical value as the insertion method being used.

<Specific Method for Computing Likelihood of Insertion Method>

As described above, for example, the determination unit 2040 computes the likelihood that the insertion method is used for each of the plurality of insertion methods in order to determine the insertion method being used. Hereinafter, as a method of computing this likelihood, two methods will be exemplified as specific examples.

<<Method Using Identification Model>>

In this method, an identification model that is trained to output the likelihood that each insertion method is being used in response to the shape data sequence 50 being input is utilized. Hereinafter, this identification model is referred to as an insertion method identification model. For example, the insertion method identification model is implemented by using an RNN which is a neural network that handles time-series data. However, the type of the model used for implementing the insertion method identification model is not limited to the RNN, and any type of model that can handle the time-series data can be used.

The training of the insertion method identification model is performed using training data including a combination of "data representing time-series change in shape of endoscope scope, and ground-truth output data". The ground-truth output data is, for example, data representing the maximum likelihood (for example, 1) for the insertion method being used and representing the minimum likelihood (for example, 0) for the other insertion methods.

Note that data other than the shape data sequence 50 may be further input to the insertion method identification model. Examples of the data other than the shape data sequence 50 include still image data or video data obtained from the camera 10, data representing the motion of the operator of the endoscope scope 40 (for example, time-series data of acceleration obtained from an acceleration sensor attached to the hand of the operator, and the like), and the like. Furthermore, in a case where the shape data sequence 50 represents the shape of the endoscope scope 40 by a shape category, three-dimensional coordinates of each of a plurality of locations of the endoscope scope 40 may be further input to the insertion method identification model. When these data are used, the same type of data is also included in the training data of the insertion method identification model. In this way, the insertion method identification model is trained to compute the likelihood by further using data other than the shape data sequence 50.

Note that the insertion method identification model may output a label representing the insertion method being used, instead of outputting the likelihood for each insertion method. In this case, for example, the insertion method identification model outputs the label representing the insertion method for which the maximum likelihood is computed as a label of the insertion method being used. The determination unit 2040 can determine the insertion method being used by the label output from the insertion method specific model.

<<Method Using State Transition Model>>

In this case, for each of the plurality of insertion methods, the state transition model representing a temporal change pattern of the shape of the endoscope scope 40 in a case where the insertion method is used is determined and stored in advance in a storage apparatus accessible from the determination unit 2040. When this method is used, the likelihood that each insertion method is used represents, for example, a degree of matching between the state transition model of the insertion method and the temporal change of the endoscope scope 40 represented by the shape data sequence 50. That is, for each of the plurality of insertion methods, the determination unit 2040 computes the degree to which the state transition model of the insertion method matches the shape data sequence 50, and determines the insertion method being used based on the computation result. For example, the insertion method with the maximum degree of matching is determined as the insertion method being used.

In a case where the shape data 52 represents the shape category, for example, the likelihood is determined as follows using the degree of matching between the state transition model and the shape data sequence 50.
Equation 1

$$likelihood = \frac{number\ of\ shape\ categories\ matching\ each\ other}{unit\ period\ length} \quad (1)$$

In Equation (1), the numerator on the right side represents the number of shape categories that match between the shape data sequence 50 and the state transition model. Meanwhile, the unit period length of the denominator represents the total number of shape categories included in the unit period described above.

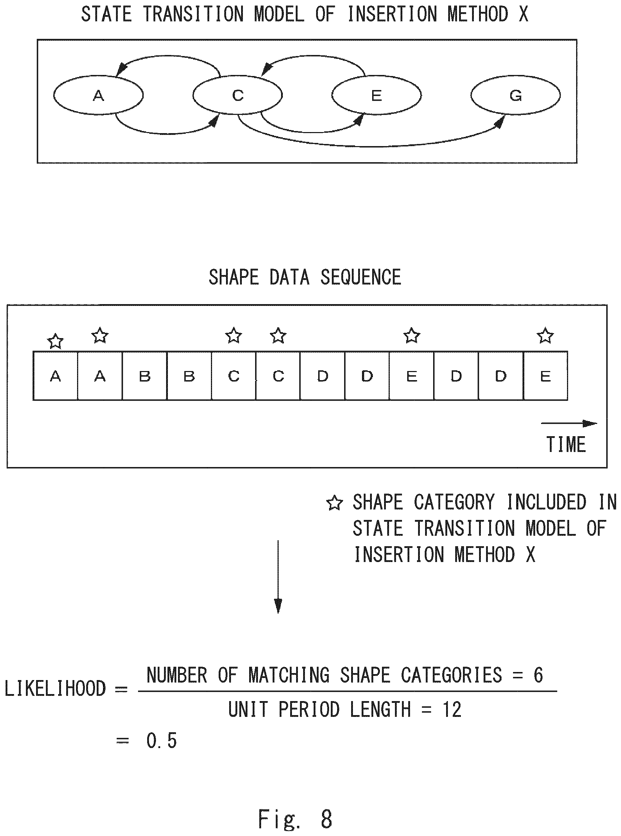

Fig. 8 is a diagram conceptually illustrating a method of calculating the likelihood using Equation (1). In this example, the state transition model of the insertion method X is compared with the shape data sequence 50. In this example, the unit period length is 12.

The state transition model of the insertion method X includes four shape categories A, C, E, and G. The shape data sequence 50 includes two shape categories A, two shape categories C, and two shape categories E among the shape categories constituting the state transition model of the insertion method X. From this, the number of shape categories that match between the state transition model of the insertion method X and the shape data sequence 50 is 6. Therefore, according to Equation (1), 0.5 (6/12) is computed as the likelihood that the insertion method X is used.

A formula for computing the likelihood is not limited to Equation (1). For example, the likelihood may be computed using the following Equation (2).
Equation 2

$$likelihood$$
$$= \frac{number\ of\ shape\ categories\ matching\ each\ other}{total\ number\ of\ types\ of\ shape\ categories\ included\ in\ unit\ peiord} \quad (2)$$

For example, in the example of Fig. 8, five types of shape categories A, B, C, D, and E are included in the shape data sequence 50 of the unit period. Meanwhile, there are three types of shape categories A, C, and E that match each other between the state transition model of the insertion method X and the shape data sequence 50. Therefore, according to Equation (2), 0.6 (3/5) is computed as the likelihood that the insertion method X is used.

Furthermore, when computing the likelihood for each insertion method, the likelihood may be computed in consideration of the order of transitions by adding a computation formula that increases the likelihood in a case of following the transition path (order relation) indicated in the state transition model or decreases the likelihood in a case of not following the transition path, in addition to the degree of matching of the shape categories included in the unit period.

<Output of Target Information 20: S108>

The output unit 2060 outputs the target information 20. There are various methods of outputting the target information 20. For example, the output unit 2060 displays a screen representing the target information 20 on a display apparatus that can be viewed by the operator of the endoscope scope 40. In another example, the output unit 2060 may store a file representing the target information 20 in a storage device or transmit the file to another arbitrary device.

The target information 20 is various types of information with which the target category can be grasped. For example, the target information 20 indicates the name of the target category and a photograph or a drawing representing the shape of the endoscope scope 40 belonging to the target category. The target information 20 in the example of Fig. 1 indicates an image representing the name of the "category E" and the shape of the endoscope scope 40 belonging to the category E with respect to the category E which is the target category.

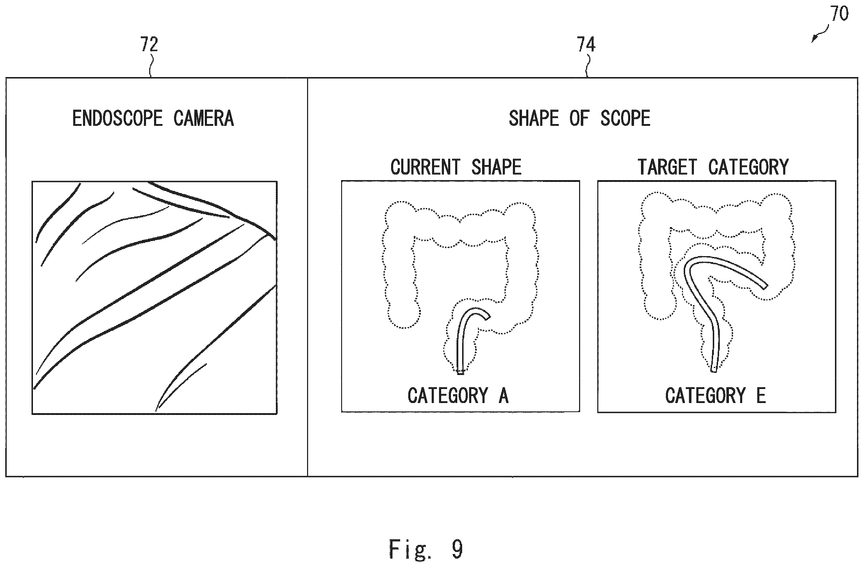

The target information 20 may be output together with information other than the information representing the target category. For example, the target information 20 is output together with an image from the camera 10 (video data obtained from the camera 10) and information representing a shape category to which the current shape of the endoscope scope 40 belongs. Fig. 9 is a diagram illustrating a screen including the target information 20. The screen 70 includes a region 72 representing an image from the camera 10 and a region 74 representing information regarding the shape of the endoscope scope 40. In the region 74, the shape category to which the shape of the current endoscope scope 40 belongs and the target category are illustrated.

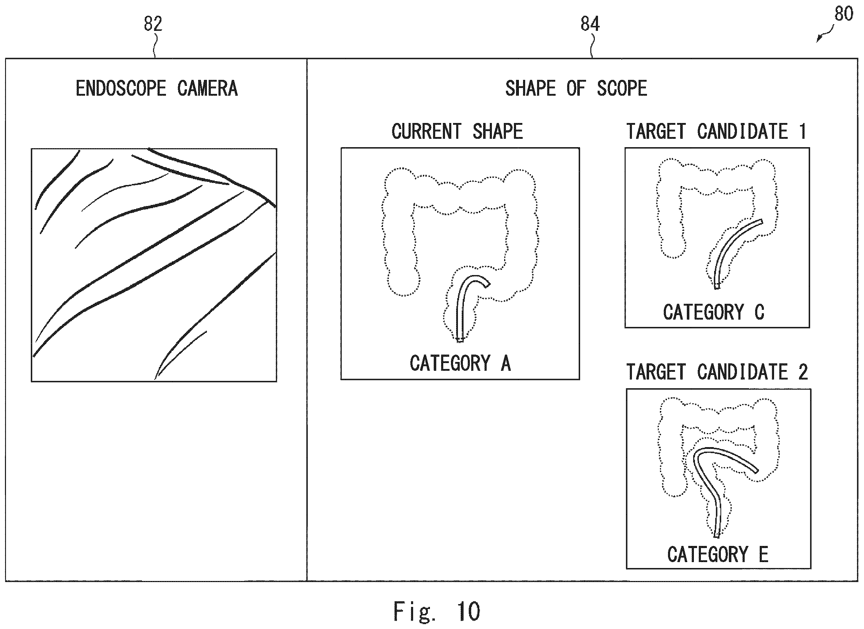

Here, the target category included in the target information 20 is not limited to one. For example, the output unit 2060 may include information regarding a plurality of candidates of the target category determined by the determination unit 2040. Fig. 10 is a diagram illustrating the target information 20 including the plurality of shape categories determined as the candidates of the target category. Similarly to the screen 70 of Fig. 9, the screen 80 includes a region 82 representing the image of the camera 10 and a region 84 representing the information regarding the shape of the endoscope scope 40.

In the example of Fig. 10, the category C and the category E are determined as the target categories by the determination unit 2040. Therefore, in the region 84, the category C and the category E are illustrated as the target categories. In addition, in each target category, the likelihood computed for the target category is indicated.

When the plurality of target categories are indicated, display modes thereof may be different from each other. For example, in a case where the plurality of target categories can be prioritized, a target category with a higher priority order may be displayed in a more emphasized manner. As a method of emphasizing, a method of increasing the size or thickening the frame can be adopted. Note that, in a case where the plurality of target categories are determined, a method of prioritizing the target categories is as described above.

The endoscope operation support apparatus 2000 may output other information in addition to or instead of the target category as the information regarding the operation to be applied to the endoscope scope 40. For example, the target information 20 may indicate the target position that is an appropriate position as the transition destination of the endoscope scope 40. For example, the determination unit 2040 determines the target position of the endoscope scope 40 based on the target category. The target position of the endoscope scope 40 is, for example, a position where the tip of the endoscope scope 40 should be reached. The output unit 2060 outputs the target information 20 representing the target position of the endoscope scope 40 in addition to or instead of the target category.

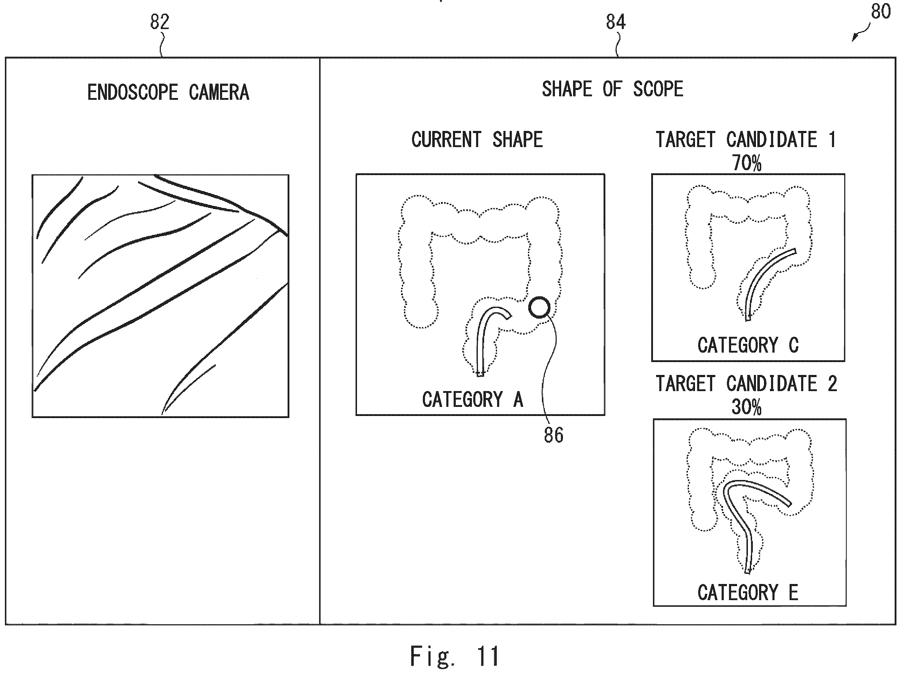

Fig. 11 is a diagram illustrating the target information 20 including the target position. In the screen 80 of Fig. 11, in the region 84, a mark 86 representing the target position is illustrated on an image representing the current shape category of the endoscope scope 40. Except for this point, the screen 80 of Fig. 11 is the same as the screen 80 of Fig. 10.

The target position of the endoscope scope 40 can be determined based on the position of the endoscope scope 40 in the target category. For example, the determination unit 2040 determines which portion of the organ that is a target of the insertion (large intestine, in this case) is the target position of the endoscope scope 40 for the shape category having the maximum likelihood among the target categories. For example, in the example of Fig. 11, a portion in which the tip of the endoscope scope 40 is located in category C is a sigmoid colon. Therefore, the determination unit 2040 displays the mark 86 in the portion of the sigmoid colon on the image representing the current situation of the endoscope scope 40.

Here, the position (for example, the portion of the organ that is the target of the insertion) to be handled as the target position of the endoscope scope 40 is determined in advance for each shape category. For example, for each shape category, the name of the shape category, the image (for example, the image of the shape category used for the screen 70 or the like) representing the shape of the endoscope scope 40 in the shape category, and information (hereinafter, category information) representing the target position of the endoscope scope 40 are stored in advance in a storage device accessible from the endoscope operation support apparatus 2000. The endoscope operation support apparatus 2000 acquires the category information from the storage device and uses the category information for generation of the target information 20.

The method of determining the target position is not limited to the method of determining based on the target category. For example, an identification model (hereinafter, the target position identification model) trained to output the target position in response to input of data representing a time-series change in the shape of the endoscope scope 40 can be used. The target position represents, for example, any of a plurality of predetermined portions in the body. The determination unit 2040 determines the target position by inputting the shape data sequence 50 to the target position identification model.

Similarly to the target category identification model, the target position identification model can be implemented by various models that can handle time-series data such as RNN. Furthermore, the target position identification model can be trained by training data configured by a combination of "data representing time-series change in shape of endoscope scope, ground-truth target position", for example.

Note that the target position identification model may be configured to output the likelihood of the target position for each predetermined position. In this case, the determination unit 2040 obtains the likelihood for each predetermined position by inputting the shape data sequence 50 to the target position identification model, and determines the target position based on the likelihood obtained for each predetermined position. For example, the determination unit 2040 treats a predetermined position having the maximum likelihood as the target position. In another example, the determination unit 2040 may treat the plurality of predetermined positions whose likelihood is equal to or more than the threshold or the plurality of predetermined positions whose order of the likelihood is less than or equal to a predetermined order as candidates of the target position and determine the target position from the candidates. As a method of determining the target position from the predetermined position of the candidate, a method similar to the method of determining the target category from the shape category of the candidate can be used.

In addition, the determination unit 2040 may treat each of the candidate predetermined positions as the target position. At this time, the priority order may be determined for these predetermined positions. As a method of determining the priority order for the predetermined position of the candidate, a method similar to the method of determining the priority order for the target category of the candidate can be used.

In the target information 20 in the previous examples, the shape of the endoscope scope 40 is represented by a plane. However, the target information 20 may represent the shape of the endoscope scope 40 in a three-dimensional manner.

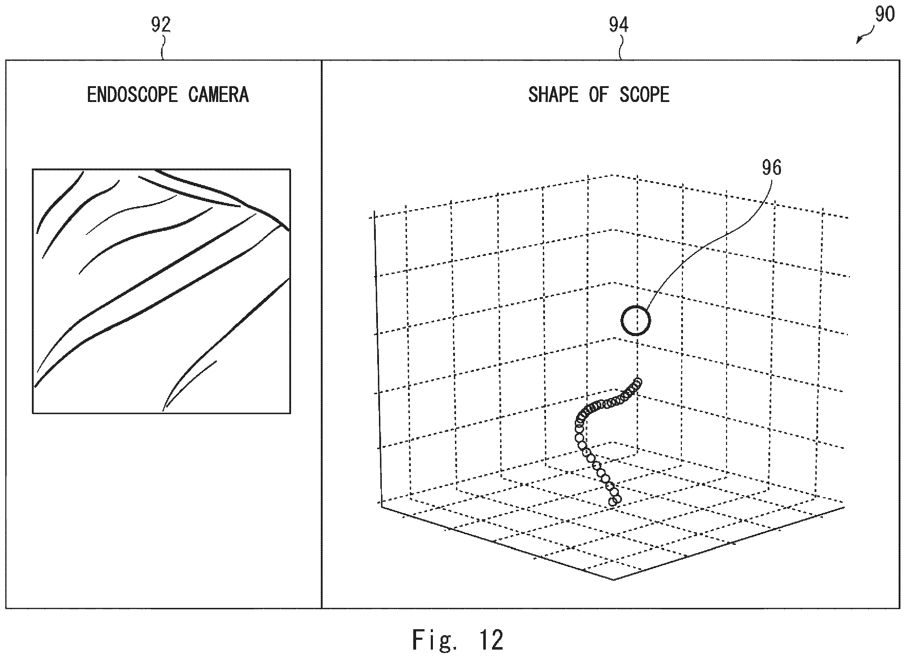

Fig. 12 is a diagram illustrating the target information 20 representing the shape of the endoscope scope 40 in a three-dimensional image. A screen 90 includes a region 92 representing the video of the camera 10 and a region 94 representing the shape of the endoscope scope 40 as a three-dimensional image. In the region 94, the shape of the endoscope scope 40 is represented by a trajectory of the position of the distal end of the endoscope scope 40. Furthermore, a mark 96 representing a target position of the endoscope scope 40 is illustrated in the region 94. Note that the three-dimensional coordinates of a specific location (for example, the tip) of the endoscope scope 40 to be set as the target position can be grasped by providing the above-described element for determining the three-dimensional coordinates of each location of the endoscope scope 40 at the specific location.

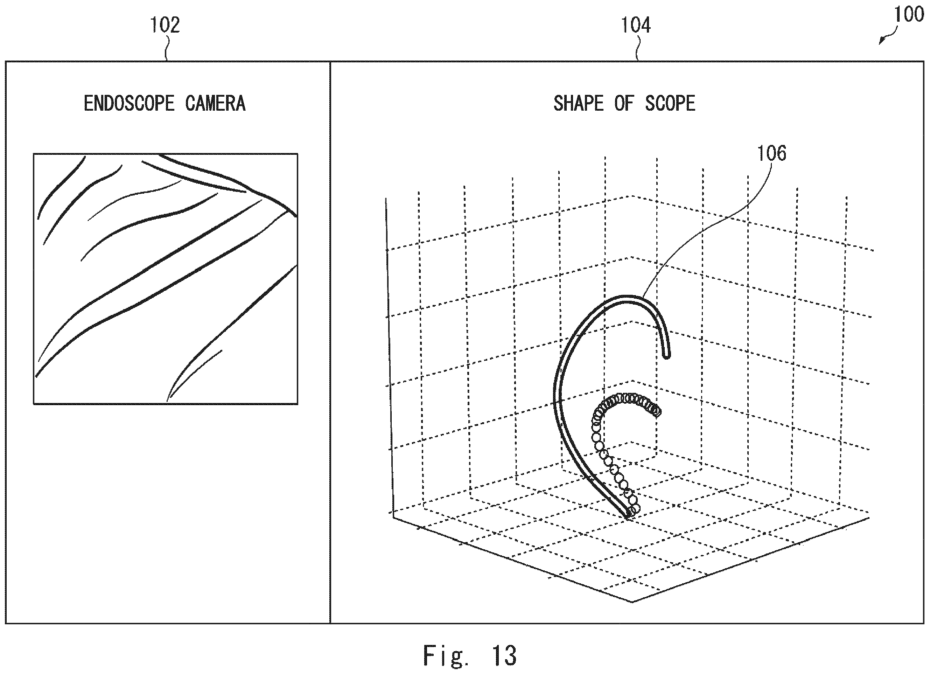

The target information 20 may indicate the shape of the endoscope scope 40 corresponding to the target category in a three-dimensional manner. Fig. 13 is a diagram illustrating the target information 20 in which the shape of the endoscope scope 40 corresponding to the target category is represented by a three-dimensional image. The screen 100 includes a region 102 representing the video from the camera 10 and a region 104 representing the shape of the endoscope scope 40 as a three-dimensional image. In the region 104, the shape of the endoscope scope 40 is represented by the trajectory of the position of the tip thereof, similarly to the region 94 of Fig. 12. Further, in the region 104, the shape of the endoscope scope 40 corresponding to the target category is represented by a three-dimensional image 106. In other words, a shape of the endoscope scope 40 to be changed is represented by the three-dimensional image 106.

Here, in order to generate the three-dimensional image 106, for each shape category, three-dimensional data (a set of three-dimensional coordinates) representing the shape of the endoscope scope 40 belonging to the shape category is required. Therefore, for example, three-dimensional data for each shape category is included in the category information described above. The output unit 2060 acquires three-dimensional data representing the shape of the endoscope scope 40 from the category information acquired for the target category determined by the determination unit 2040, and generates the three-dimensional image 106 using the three-dimensional data.

However, the three-dimensional coordinates of each location of the endoscope scope 40 in a certain shape may vary depending on a size of a body of a person into which the endoscope scope 40 is inserted (Even when the figures of the trajectory of the tip of the endoscope scope 40 are similar, they are not congruent). Therefore, for example, the determination unit 2040 generates the three-dimensional image 106 of the target category by performing similarity transformation of a figure represented by the three-dimensional data corresponding to the target category in accordance with a size of a trajectory of a tip position of the endoscope scope 40. For example, the determination unit 2040 acquires the three-dimensional data corresponding to the shape category to which the current shape of the endoscope scope 40 belongs, and computes the ratio of the size of the trajectory of the endoscope scope 40 to the size of the figure represented by the three-dimensional data prepared in advance by comparing the three-dimensional data with the trajectory of the tip of the endoscope scope 40. Then, the output unit 2060 generates the three-dimensional image 106 of the target category by performing similarity transformation on the size of the figure represented by the three-dimensional data corresponding to the target category by the ratio.

The target information 20 may further include information used for determining the target category and the target position. For example, the name of the currently used insertion method and a diagram of the state transition model of the insertion method may be included in the target information 20. Furthermore, in a case where the determination unit 2040 determines that it is necessary to change the insertion method, the target information 20 may include the name of the changed insertion method and the diagram of the state transition model of the changed insertion method.

Although the present invention has been described above with reference to the example embodiments, the present invention is not limited to the above example embodiments. Various modifications that can be understood by those skilled in the art can be made to the configuration and details of the present invention within the scope of the present invention.

In the above example, the program can be stored using various types of non-transitory computer readable medium and provided to the computer. Non-transitory computer readable medium includes various types of tangible storage media. Examples of the non-transitory computer readable medium include a magnetic recording medium (for example, a flexible disk, a magnetic tape, or a hard disk drive), a magneto-optical recording medium (for example, a magneto-optical disk), a CD-ROM, a CD-R, a CD-R/W, and a semiconductor memory (for example, mask ROM, PROM (Programmable ROM), EPROM (Erasable PROM), flash ROM, and RAM). In addition, the program may be provided to the computer by various types of transitory computer readable medium. Examples of transitory computer readable medium include electrical signals, optical signals, and electromagnetic waves. The transitory computer readable medium can supply the program to the computer via a wired communication path such as an electric wire and an optical fiber, or a wireless communication path.

Some or all of the above example embodiments may be described as the following supplementary notes, but are not limited to the following.

(Supplementary Note 1)

An endoscope operation support apparatus comprising:

an acquisition unit configured to acquire a shape data sequence representing a temporal change of a shape category of an endoscope scope inserted into a body;
a determination unit configured to determine a target category suitable as a transition destination of the shape category of the endoscope scope or a target position which is a position suitable as a position at which the endoscope scope transitions based on the shape data sequence; and
an output unit configured to output target information that is information regarding the target category or the target position.

(Supplementary Note 2)

The endoscope operation support apparatus according to Supplementary note 1, wherein the determination unit determines the target category or the target position by inputting the shape data sequence to a trained identification model configured to output the target category or the target position in response to an input of a data sequence representing a temporal change of a shape of the endoscope scope.

(Supplementary Note 3)

The endoscope operation support apparatus according to Supplementary note 1, wherein the determination unit acquires a state transition model corresponding to a currently used insertion method from state transition models representing a time transition pattern of a shape category of the endoscope scope defined for each of a plurality of insertion methods of the endoscope scope, and determines the target category using the acquired state transition model.

(Supplementary Note 4)

The endoscope operation support apparatus according to Supplementary note 3, wherein the determination unit determines a last shape category in the shape data sequence from the acquired state transition model, and determines the shape category to which it is possible to transition from the determined shape category in the acquired state transition model as the target category.

(Supplementary Note 5)

The endoscope operation support apparatus according to Supplementary note 3, wherein the determination unit performs:

determining whether or not it is necessary to change an insertion method of the endoscope scope;
determining an insertion method after a change in a case where it is necessary to change the insertion method of the endoscope scope; and
determining the target category using the state transition model corresponding to the changed insertion method.

(Supplementary Note 6)

The endoscope operation support apparatus according to Supplementary note 5, wherein the determination unit determines whether or not it is necessary to change the insertion method of the endoscope scope based on information regarding an operator of the endoscope scope.

(Supplementary Note 7)

The endoscope operation support apparatus according to any one of Supplementary notes 1 to 6, wherein the determination unit performs:

determining a plurality of candidates of the target category or a candidate of a candidate of the target position; and determining the target category or the target position from a plurality of candidates based on a priority determined for each shape category or each position.

(Supplementary Note 8)

The endoscope operation support apparatus according to any one of Supplementary notes 1 to 7, wherein a priority of each shape category or each position is determined based on the insertion method of the endoscope scope or information regarding an operator of the endoscope scope.

(Supplementary Note 9)

The endoscope operation support apparatus according to any one of Supplementary notes 1 to 8, wherein the target information includes information representing a current shape category of the endoscope scope.

(Supplementary Note 10)

The endoscope operation support apparatus according to Supplementary note 9, wherein the target information includes an image of an inside of the body on which an image representing a current shape of the endoscope scope and an image representing the target position are superimposed.

(Supplementary Note 11)

The endoscope operation support apparatus according to any one of Supplementary notes 1 to 10, wherein the target information indicates a plurality of candidates of the target category or the target position together with likelihood that each candidate is the target category or the target position.

(Supplementary Note 12)

A control method executed by a computer, the control method comprising:

an acquisition step of acquiring a shape data sequence representing a temporal change of a shape category of an endoscope scope inserted into a body; a determining step of determining a target category suitable as a transition destination of the shape category of the endoscope scope or a target position which is a position suitable as a position at which the endoscope scope transitions based on the shape data sequence; and an output step of outputting target information that is information regarding the target category or the target position.

(Supplementary Note 13)

The control method according to Supplementary note 12, wherein in the determining step, the target category or the target position is determined by inputting the shape data sequence to a trained identification model configured to output the target category or the target position in response to an input of a data sequence representing a temporal change of a shape of the endoscope scope.

(Supplementary Note 14)

The control method according to Supplementary note 12, wherein in the determining step, the state transition model corresponding to a currently used insertion method is acquired from state transition models representing a time transition pattern of a shape category of the endoscope scope defined for each of a plurality of insertion methods of the endoscope scope, and the target category is determined using the acquired state transition model.

(Supplementary Note 15)

The control method according to Supplementary note 14, wherein in the determining step, a last shape category in the shape data sequence is determined from the acquired state transition model, and the shape category to which it is possible to transition from the determined shape category in the acquired state transition model is determined as the target category.

(Supplementary Note 16)

The control method according to Supplementary note 14, wherein in the determining step,

whether or not it is necessary to change the insertion method of the endoscope scope is determined,
an insertion method after a change is determined in a case where it is necessary to change the insertion method of the endoscope scope, and
the target category is determined using the state transition model corresponding to the changed insertion method.

(Supplementary Note 17)

The control method according to Supplementary note 16, wherein in the determining step, it is determined whether or not it is necessary to change the insertion method of the endoscope scope based on information regarding an operator of the endoscope scope.

(Supplementary Note 18)

The control method according to any one of Supplementary notes 12 to 17, wherein in the determining step,

a plurality of candidates of the target category or a candidate of a candidate of the target position are determined, and
the target category or the target position is determined from a plurality of candidates based on a priority determined for each shape category or each position.

(Supplementary Note 19)

The control method according to any one of Supplementary notes 12 to 18, wherein a priority of each shape category or each position is determined based on an insertion method of the endoscope scope or information regarding an operator of the endoscope scope.

(Supplementary Note 20)

The control method according to any one of Supplementary notes 12 to 19, wherein the target information includes information representing a current shape category of the endoscope scope.

(Supplementary Note 21)

The control method according to Supplementary note 20, wherein the target information includes an image of an inside of the body on which an image representing a current shape of the endoscope scope and an image representing the target position are superimposed.

(Supplementary Note 22)

The control method according to any one of Supplementary notes 12 to 21, wherein the target information indicates a plurality of candidates of the target category or the target position together with likelihood that each candidate is the target category or the target position.

(Supplementary Note 23)

A computer readable medium storing a program, the program causing a computer to execute:

an acquisition step of acquiring a shape data sequence representing a temporal change of a shape category of an endoscope scope inserted into a body;

a determining step of determining a target category suitable as a transition destination of the shape category of the endoscope scope or a target position which is a position suitable as a position at which the endoscope scope transitions based on the shape data sequence; and

an output step of outputting target information that is information regarding the target category or the target position.

(Supplementary Note 24)

The computer readable medium according to Supplementary note 23, wherein in the determining step, the target category or the target position is determined by inputting the shape data sequence to a trained identification model configured to output the target category or the target position in response to an input of a data sequence representing a temporal change of a shape of the endoscope scope.

(Supplementary Note 25)

The computer readable medium according to Supplementary note 23, wherein in the determining step, the state transition model corresponding to a currently used insertion method is acquired from state transition models representing a time transition pattern of a shape category of the endoscope scope defined for each of a plurality of insertion methods of the endoscope scope, and the target category is determined using the acquired state transition model.

(Supplementary Note 26)

The computer readable medium according to Supplementary note 25, wherein in the determining step, a last shape category in the shape data sequence is determined from the acquired state transition model, and the shape category to which it is possible to transition from the determined shape category in the acquired state transition model is determined as the target category.

(Supplementary Note 27)

The computer readable medium according to Supplementary note 25, wherein in the determining step,

whether or not it is necessary to change the insertion method of the endoscope scope is determined,
an insertion method after a change is determined in a case where it is necessary to change the insertion method of the endoscope scope, and
the target category is determined using the state transition model corresponding to the changed insertion method.

(Supplementary Note 28)

The computer readable medium according to Supplementary note 27, wherein, in the determining step, it is determined whether or not it is necessary to change the insertion method of the endoscope scope based on information regarding an operator of the endoscope scope.

(Supplementary Note 29)

The computer readable medium according to any one of Supplementary notes 23 to 28, wherein in the determining step,

a plurality of candidates of the target category or a candidate of a candidate of the target position are determined, and
the target category or the target position is determined from a plurality of candidates based on a priority determined for each shape category or each position.

(Supplementary Note 30)

The computer readable medium according to any one of Supplementary notes 23 to 29, wherein a priority of each shape category or each position is determined based on the insertion method of the endoscope scope or information regarding the operator of the endoscope scope.

(Supplementary Note 31)

The computer readable medium according to any one of Supplementary notes 23 to 30, wherein the target information includes information representing the current shape category of the endoscope scope.

(Supplementary Note 32)

The computer readable medium according to Supplementary note 31, wherein the target information includes an image of an inside of the body on which an image representing the current shape of the endoscope scope and an image representing the target position are superimposed.

(Supplementary Note 33)

The computer readable medium according to any one of Supplementary notes 23 to 32, wherein the target information indicates a plurality of candidates of the target category or the target position together with likelihood that each candidate is the target category or the target position.

(Supplementary Note 34)

A program causing a computer to execute:

an acquisition step of acquiring a shape data sequence representing a temporal change of a shape category of an endoscope scope inserted into a body;
a determining step of determining a target category suitable as a transition destination of the shape category of the endoscope scope or a target position which is a position suitable as a position at which the endoscope scope transitions based on the shape data sequence; and
an output step of outputting target information that is information regarding the target category or the target position.

(Supplementary Note 35)

The program according to Supplementary note 34, wherein in the determining step, the target category or the target position is determined by inputting the shape data sequence to a trained identification model configured to output the target category or the target position in response to an input of a data sequence representing a temporal change of a shape of the endoscope scope.

(Supplementary Note 36)

The program according to Supplementary note 34, wherein in the determining step, the state transition model corresponding to a currently used insertion method is acquired from state transition models representing a time transition pattern of a shape category of the endoscope scope defined for each of a plurality of insertion methods of the endoscope scope, and the target category is determined using the acquired state transition model.

(Supplementary Note 37)

The program according to Supplementary note 36, wherein in the determining step, a last shape category in the shape data sequence is determined from the acquired state transition model, and the shape category to which it is possible to transition from the determined shape category in the acquired state transition model is determined as the target category.

(Supplementary Note 38)

The program according to Supplementary note 36, wherein in the determining step,

whether or not it is necessary to change the insertion method of the endoscope scope is determined,
an insertion method after a change is determined in a case where it is necessary to change the insertion method of the endoscope scope, and
the target category is determined using the state transition model corresponding to the changed insertion method.

(Supplementary Note 39)

The program according to Supplementary note 38, wherein, in the determining step, it is determined whether or not it is necessary to change the insertion method of the endoscope scope based on information regarding an operator of the endoscope scope.

(Supplementary Note 40)

The program according to any one of Supplementary notes 34 to 39, wherein in the determining step,

a plurality of candidates of the target category or a candidate of a candidate of the target position are determined, and the target category or the target position is determined from a plurality of candidates based on a priority determined for each shape category or each position.

(Supplementary Note 41)

The program according to any one of Supplementary notes 34 to 40, wherein a priority of each shape category or each position is determined based on the insertion method of the endoscope scope or information regarding the operator of the endoscope scope.

(Supplementary Note 42)

The program according to any one of Supplementary notes 34 to 41, wherein the target information includes information representing the current shape category of the endoscope scope.

(Supplementary Note 43)

The program according to Supplementary note 42, wherein the target information includes an image of an inside of the body on which an image representing the current shape of the endoscope scope and an image representing the target position are superimposed.

(Supplementary Note 44)

The program according to any one of Supplementary notes 34 to 43, wherein the target information indicates a plurality of candidates of the target category or the target position together with likelihood that each candidate is the target category or the target position.

**Reference Signs List**

| | |
|---|---|
| 10 | CAMERA |
| 20 | TARGET INFORMATION |
| 40 | ENDOSCOPE SCOPE |
| 50 | SHAPE DATA SEQUENCE |
| 51 | TIME POINT |
| 52 | SHAPE DATA |
| 60 | ENDOSCOPE CONTROL APPARATUS |
| 70 | SCREEN |
| 72 | REGION |
| 74 | REGION |
| 80 | SCREEN |
| 82 | REGION |
| 84 | REGION |
| 86 | MARK |
| 90 | SCREEN |
| 92 | REGION |
| 94 | REGION |

| 96 | MARK |
| 100 | SCREEN |
| 102 | REGION |
| 104 | REGION |
| 106 | THREE-DIMENSIONAL IMAGE |
| 500 | COMPUTER |
| 502 | BUS |
| 504 | PROCESSOR |
| 506 | MEMORY |
| 508 | STORAGE DEVICE |
| 510 | INPUT/OUTPUT INTERFACE |
| 512 | NETWORK INTERFACE |
| 2000 | ENDOSCOPE OPERATION SUPPORT APPARATUS |
| 2020 | ACQUISITION UNIT |
| 2040 | DETERMINATION UNIT |
| 2060 | OUTPUT UNIT |

**Claims**

1. An endoscope operation support apparatus comprising:

   an acquisition unit configured to acquire a shape data sequence representing a temporal change of a shape category of an endoscope scope inserted into a body;
   a determination unit configured to determine a target category suitable as a transition destination of the shape category of the endoscope scope or a target position which is a position suitable as a position at which the endoscope scope transitions based on the shape data sequence; and
   an output unit configured to output target information that is information regarding the target category or the target position.

2. The endoscope operation support apparatus according to Claim 1, wherein the determination unit determines the target category or the target position by inputting the shape data sequence to a trained identification model configured to output the target category or the target position in response to an input of a data sequence representing a temporal change of a shape of the endoscope scope.

3. The endoscope operation support apparatus according to Claim 1, wherein the determination unit acquires a state transition model corresponding to a currently used insertion method from state transition models representing a time transition pattern of a shape category of the endoscope scope defined for each of a plurality of insertion methods of the endoscope scope, and determines the target category using the acquired state transition model.

4. The endoscope operation support apparatus according to Claim 3, wherein the determination unit determines a last shape category in the shape data sequence from the acquired state transition model, and determines the shape category to which it is possible to transition from the determined shape category in the acquired state transition model as the target category.

5. The endoscope operation support apparatus according to Claim 3, wherein the determination unit performs:

   determining whether or not it is necessary to change an insertion method of the endoscope scope;
   determining an insertion method after a change in a case where it is necessary to change the insertion method of the endoscope scope; and
   determining the target category using the state transition model corresponding to the changed insertion method.

6. The endoscope operation support apparatus according to Claim 5, wherein the determination unit determines whether or not it is necessary to change the insertion method of the endoscope scope based on information regarding an operator of the endoscope scope.

7. The endoscope operation support apparatus according to any one of Claims 1 to 6, wherein the determination unit performs:

0

0

determining a plurality of candidates of the target category or a candidate of a candidate of the target position; and determining the target category or the target position from a plurality of candidates based on a priority determined for each shape category or each position.

8. The endoscope operation support apparatus according to any one of Claims 1 to 7, wherein a priority of each shape category or each position is determined based on the insertion method of the endoscope scope or information regarding an operator of the endoscope scope.

9. The endoscope operation support apparatus according to any one of Claims 1 to 8, wherein the target information includes information representing a current shape category of the endoscope scope.

10. The endoscope operation support apparatus according to Claim 9, wherein the target information includes an image of an inside of the body on which an image representing a current shape of the endoscope scope and an image representing the target position are superimposed.

11. The endoscope operation support apparatus according to any one of Claims 1 to 10, wherein the target information indicates a plurality of candidates of the target category or the target position together with likelihood that each candidate is the target category or the target position.

12. A control method executed by a computer, the control method comprising:

an acquisition step of acquiring a shape data sequence representing a temporal change of a shape category of an endoscope scope inserted into a body;
a determining step of determining a target category suitable as a transition destination of the shape category of the endoscope scope or a target position which is a position suitable as a position at which the endoscope scope transitions based on the shape data sequence; and
an output step of outputting target information that is information regarding the target category or the target position.

13. The control method according to Claim 12, wherein in the determining step, the target category or the target position is determined by inputting the shape data sequence to a trained identification model configured to output the target category or the target position in response to an input of a data sequence representing a temporal change of a shape of the endoscope scope.

14. The control method according to Claim 12, wherein in the determining step, the state transition model corresponding to a currently used insertion method is acquired from state transition models representing a time transition pattern of a shape category of the endoscope scope defined for each of a plurality of insertion methods of the endoscope scope, and the target category is determined using the acquired state transition model.

15. The control method according to Claim 14, wherein in the determining step, a last shape category in the shape data sequence is determined from the acquired state transition model, and the shape category to which it is possible to transition from the determined shape category in the acquired state transition model is determined as the target category.

16. The control method according to Claim 14, wherein in the determining step,

whether or not it is necessary to change the insertion method of the endoscope scope is determined,
an insertion method after a change is determined in a case where it is necessary to change the insertion method of the endoscope scope, and
the target category is determined using the state transition model corresponding to the changed insertion method.

17. The control method according to Claim 16, wherein in the determining step, it is determined whether or not it is necessary to change the insertion method of the endoscope scope based on information regarding an operator of the endoscope scope.

18. The control method according to any one of Claims 12 to 17, wherein in the determining step,

a plurality of candidates of the target category or a candidate of a candidate of the target position are determined, and

the target category or the target position is determined from a plurality of candidates based on a priority determined for each shape category or each position.

19. The control method according to any one of Claims 12 to 18, wherein a priority of each shape category or each position is determined based on an insertion method of the endoscope scope or information regarding an operator of the endoscope scope.

20. The control method according to any one of Claims 12 to 19, wherein the target information includes information representing a current shape category of the endoscope scope.

21. The control method according to Claim 20, wherein the target information includes an image of an inside of the body on which an image representing a current shape of the endoscope scope and an image representing the target position are superimposed.

22. The control method according to any one of Claims 12 to 21, wherein the target information indicates a plurality of candidates of the target category or the target position together with likelihood that each candidate is the target category or the target position.

23. A computer readable medium storing a program, the program causing a computer to execute:

an acquisition step of acquiring a shape data sequence representing a temporal change of a shape category of an endoscope scope inserted into a body;
a determining step of determining a target category suitable as a transition destination of the shape category of the endoscope scope or a target position which is a position suitable as a position at which the endoscope scope transitions based on the shape data sequence; and
an output step of outputting target information that is information regarding the target category or the target position.

24. The computer readable medium according to Claim 23, wherein in the determining step, the target category or the target position is determined by inputting the shape data sequence to a trained identification model configured to output the target category or the target position in response to an input of a data sequence representing a temporal change of a shape of the endoscope scope.

25. The computer readable medium according to Claim 23, wherein in the determining step, the state transition model corresponding to a currently used insertion method is acquired from state transition models representing a time transition pattern of a shape category of the endoscope scope defined for each of a plurality of insertion methods of the endoscope scope, and the target category is determined using the acquired state transition model.

26. The computer readable medium according to Claim 25, wherein in the determining step, a last shape category in the shape data sequence is determined from the acquired state transition model, and the shape category to which it is possible to transition from the determined shape category in the acquired state transition model is determined as the target category.

27. The computer readable medium according to Claim 25, wherein in the determining step,

whether or not it is necessary to change the insertion method of the endoscope scope is determined,
an insertion method after a change is determined in a case where it is necessary to change the insertion method of the endoscope scope, and
the target category is determined using the state transition model corresponding to the changed insertion method.

28. The computer readable medium according to Claim 27, wherein, in the determining step, it is determined whether or not it is necessary to change the insertion method of the endoscope scope based on information regarding an operator of the endoscope scope.

29. The computer readable medium according to any one of Claims 23 to 28, wherein in the determining step,

a plurality of candidates of the target category or a candidate of a candidate of the target position are determined, and

the target category or the target position is determined from a plurality of candidates based on a priority determined for each shape category or each position.

30. The computer readable medium according to any one of Claims 23 to 29, wherein a priority of each shape category or each position is determined based on the insertion method of the endoscope scope or information regarding the operator of the endoscope scope.

31. The computer readable medium according to any one of Claims 23 to 30, wherein the target information includes information representing the current shape category of the endoscope scope.

32. The computer readable medium according to Claim 31, wherein the target information includes an image of an inside of the body on which an image representing the current shape of the endoscope scope and an image representing the target position are superimposed.

33. The computer readable medium according to any one of Claims 23 to 32, wherein the target information indicates a plurality of candidates of the target category or the target position together with likelihood that each candidate is the target category or the target position.

34. A program causing a computer to execute:

an acquisition step of acquiring a shape data sequence representing a temporal change of a shape category of an endoscope scope inserted into a body;
a determining step of determining a target category suitable as a transition destination of the shape category of the endoscope scope or a target position which is a position suitable as a position at which the endoscope scope transitions based on the shape data sequence; and
an output step of outputting target information that is information regarding the target category or the target position.

SHAPE DATA
SEQUENCE

52

50

40

CATEGORY A          CATEGORY C

2000

ENDOSCOPE OPERATION
SUPPORT APPARATUS

DETERMINE SHAPE CATEGORY (TARGET CATEGORY) SUITABLE AS
TRANSITION DESTINATION OR POSITION (TARGET POSITION)
SUITABLE AS TRANSITION DESTINATION BASED ON TEMPORAL
CHANGE OF SHAPE CATEGORY OF ENDOSCOPE SCOPE 40

OUTPUT TARGET INFORMATION 20 REGARDING
TARGET CATEGORY OR TARGET POSITION

20

NEXT, CHANGE TO [CATEGORY E]

CATEGORY E

TARGET INFORMATION

Fig. 1

2000

ENDOSCOPE OPERATION SUPPORT APPARATUS

2020

ACQUISITION UNIT

2040

DETERMINATION UNIT

2060

OUTPUT UNIT

Fig. 2

```
                        60
                         )
              ┌──────────────────────┐
              │  ENDOSCOPE CONTROL   │                        500
              │      APPARATUS       │                         )
              └──────────────────────┘              COMPUTER
       506                        510
        )                          )
 ┌──────────────┐        ┌──────────────────┐
 │    MEMORY    │        │   INPUT/OUTPUT   │        502
 │              │        │       I/F        │         )
 └──────────────┘        └──────────────────┘
         │                        │
 ┌───────────────────────────────────────────────────────────┐
 │                           BUS                             │
 └───────────────────────────────────────────────────────────┘
         │                │                        │
 ┌──────────────┐   ┌──────────────┐      ┌──────────────────┐
 │  PROCESSOR   │   │   STORAGE    │      │   NETWORK I/F    │
 │              │   │   DEVICE     │      │                  │
 └──────────────┘   └──────────────┘      └──────────────────┘
        )                 )                        )
       504               508                      512
```

Fig. 3

40

SCOPE

CAMERA — 10

60

ENDOSCOPE CONTROL
APPARATUS

2000

ENDOSCOPE OPERATION
SUPPORT APPARATUS

Fig. 4

start

ACQUIRE SHAPE DATA SEQUENCE 50 ~S102

DETERMINE TARGET CATEGORY OR TARGET POSITION ~S104

OUTPUT TARGET INFORMATION 20 ~S106

end

Fig. 5

50

| 52 | 54 |
|---|---|
| TIME POINT | SHAPE DATA |
| t1 | CATEGORY E |
| t2 | CATEGORY B |
| . . . | . . . |

Fig. 6

STATE TRANSITION MODEL OF INSERTION METHOD X
USED TO INSERT ENDOSCOPE SCOPE 40

LAST STATE IN SHAPE
DATA SEQUENCE 50

Fig. 7

STATE TRANSITION MODEL OF INSERTION METHOD X

SHAPE DATA SEQUENCE

TIME

☆ SHAPE CATEGORY INCLUDED IN
STATE TRANSITION MODEL OF
INSERTION METHOD X

$$\text{LIKELIHOOD} = \frac{\text{NUMBER OF MATCHING SHAPE CATEGORIES} = 6}{\text{UNIT PERIOD LENGTH} = 12}$$

$$= 0.5$$

Fig. 8

ENDOSCOPE CAMERA

SHAPE OF SCOPE

CURRENT SHAPE

TARGET CATEGORY

CATEGORY A

CATEGORY E

Fig. 9

EP 4 233 676 A1

Fig. 10

| ENDOSCOPE CAMERA | SHAPE OF SCOPE |

CURRENT SHAPE

CATEGORY A

86

TARGET CANDIDATE 1
70%

CATEGORY C

TARGET CANDIDATE 2
30%

CATEGORY E

82    84    80

36

EP 4 233 676 A1

Fig. 11

ENDOSCOPE CAMERA

SHAPE OF SCOPE

Fig. 12

EP 4 233 676 A1

Fig. 13

**INTERNATIONAL SEARCH REPORT**

| International application No. |
| --- |
| PCT/JP2020/039522 |

A. CLASSIFICATION OF SUBJECT MATTER
Int. Cl. A61B1/00(2006.01)i
FI: A61B1/00 552

According to International Patent Classification (IPC) or to both national classification and IPC

B. FIELDS SEARCHED

Minimum documentation searched (classification system followed by classification symbols)
Int. Cl. A61B1/00-1/32, G02B23/24-23/26

Documentation searched other than minimum documentation to the extent that such documents are included in the fields searched
Published examined utility model applications of Japan    1922-1996
Published unexamined utility model applications of Japan  1971-2020
Registered utility model specifications of Japan          1996-2020
Published registered utility model applications of Japan  1994-2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | WO 2007/023631 A1 (OLYMPUS MEDICAL SYSTEMS CORP.) 01 March 2007 (2007-03-01), paragraphs [0001]–[0219], fig. 1-34 | 1-34 |
| A | WO 2018/207537 A1 (OLYMPUS CORP.) 15 November 2018 (2018-11-15), paragraphs [0001]-[0104], fig. 1-16 | 1-34 |
| A | WO 2019/008726 A1 (OLYMPUS CORP.) 10 January 2019 (2019-01-10), paragraphs [0001]-[0091], fig. 1-12 | 1-34 |
| A | WO 2016/207973 A1 (OLYMPUS CORP.) 29 December 2016 (2016-12-29), paragraphs [0001]-[0058], fig. 1-9 | 1-34 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 14.12.2020 | 28.12.2020 |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

INTERNATIONAL SEARCH REPORT
Information on patent family members

International application No.

PCT/JP2020/039522

| Patent Documents referred to in the Report | Publication Date | Patent Family | Publication Date |
|---|---|---|---|
| WO 2007/023631 A1 | 01.03.2007 | US 2009/0149703 A1 paragraphs [0001]- [0269], fig. 1-34 EP 1917901 A1 CN 101247752 A | |
| WO 2018/207537 A1 | 15.11.2018 | US 2020/0069149 A1 paragraphs [0001]- [0134], fig. 1-16 CN 110799085 A | |
| WO 2019/008726 A1 | 10.01.2019 | US 2020/0129043 A1 paragraphs [0001]- [0105], fig. 1-12 CN 110831476 A | |
| WO 2016/207973 A1 | 29.12.2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019097661 A